# EUROPEAN PATENT SPECIFICATION

(11) **EP 2 112 155 B1**
(45) Date of publication and mention of the grant of the patent: **29.09.2010**
(21) Application number: 08155220.0
(22) Date of filing: 25.04.2008
(51) Int. Cl.: C07D 495/04, A61K 31/4365, A61P 7/02

(54) **Hydrogensulfate salt of 2-acetoxy-5-(a-cyclopropylcarbonyl-2-fluorobenzyl)-4,5,6,7-tetrahydrothieno[3,2-c]pyridine and its preparation**
Hydrogensulfat von 2-Acetoxy-5-(a-cyclopropylcarbonyl-2-fluorbenzyl)-4,5,6,7-tetrahydrothieno[3,2-c]pyridin und dessen Zubereitung
Sel hydrogénosulfate de 2-acétoxy-5-(a-cyclopropylcarbonyl-2-fluorobenzyl)-4,5,6,7-tétrahydrothiéno[3,2-c]pyridine et sa préparation

(43) Date of publication of application: 28.10.2009
(73) Proprietor: Sandoz AG, 4056 Basel (CH)
(72) Inventor: Hotter, Andreas, 6250 Kundl (AT); Wieser, Josef, 6250 Kundl (AT); Pichler, Arthur, 6250 Kundl (AT)
(74) Representative: Wichmann, Hendrik

(56) References cited:
- WO-A-2007/114526
- US-B2- 6 693 115

## Description

### FIELD OF THE INVENTION

The present invention relates to crystalline form I of the hydrogensulfate salt of 2-acetoxy-5-(α-cyclopropylcarbonyl-2-fluorobenzyl)-4,5,6,7-tetrahydrothieno[3,2-c]pyridine. A process for the preparation of the salt, pharmaceutical compositions comprising the salt and the use of the salt as a pharmaceutical, in particular as a blood platelet aggregation inhibitor are also described. Seed crystals which can be employed in the above mentioned process as well as a process for their preparation are also disclosed.

### BACKGROUND OF THE INVENTION

Prasugrel, 2-acetoxy-5-(a-cyclopropylcarbonyl-2-fluorobenzyl)-4,5,6,7-tetrahydrothieno[3,2-c]pyridine, is a thienopyridine derivative and acts as an antiplatelet agent. The platelet activation and subsequent platelet aggregation play an essential role in the pathogenesis of cardiovascular diseases. A former clinical study could demonstrate that Prasugrel is orally active and produces a potent antiplatelet and antithrombotic action with a rapid onset and long duration *via* platelet ADP receptors antagonisms. Prasugrel is a prodrug, which means it generates an active metabolite *in vivo* (Sugidachi A., Asai F., Ogawa T., et al., "The in vivo pharmacological profile of CS-747, a novel antiplatelet agent with platelet ADP receptor antagonist properties", Br. J. Pharmacol. 2000, 129:1439-1446).

US 6,693,115 claims that acid addition salts of Prasugrel are useful as therapeutic or prophylactic agents for thrombus formation-induced or embolization-induced diseases. Prasugrel hydrochloride and Prasugrel maleate are disclosed in US 6,693,115. Furthermore a Prasugrel besylate is mentioned in WO 2007/114526.

Polymorphism is a phenomenon relating to the occurrence of different crystal forms for one molecule. There may be several different crystalline forms for the same molecule with distinct crystal structures and varying in physical properties like melting point, XRPD spectrum and IR-spectrum. These polymorphs are thus distinct solid forms which share the molecular formula of the compound from which the crystals are made up, however they may have distinct advantageous physical properties which can have a direct effect on the ability to process and/or manufacture the drug substance, like flowability, and the drug product, like flowability, as well as on drug product stability, dissolution, and bioavailability.

There remains a need for alternative acid addition salts of Prasugrel having improved physicochemical properties.

Apart from permeability, solubility is the main criterion in the biopharmaceutical classification system. An optimal active pharmaceutical ingredient for oral application should show high solubility in the range from about pH 1.0 up to about pH 8.0, in order to show high bioavailability. Therefore it is desirable to have an acid addition salt of Prasugrel with suitable solubility in a broad pH-range.

### SUMMARY OF THE INVENTION

In one embodiment, the present invention refers to crystalline form I of Prasugrel hydrogensulfate or a solvate or a hydrate thereof.

Crystalline form I of Prasugrel hydrogensulfate can be described by an X-ray powder diffraction pattern comprising peaks at 2-theta angles of 9.2 ± 0.2°, 13.1 ± 0.2°, 13.9 ± 0.2°, 14.8±0.2°, 16.0±0.2°, 17.0±0.2°, 17.7±0.2°, 18.9±0.2°, 19.7±0.2°,21.2±0.2°,22.7± 0.2°,25.1 ± 0.2° and 28.0 ± 0.2°.

Alternatively crystalline form I of Prasugrel hydrogensulfate can be described by an infrared spectrum comprising peaks at wavenumbers of 1751 ± 2 cm⁻¹, 1712 ± 2 cm⁻¹, 1495 ± 2 cm⁻¹, 1153 ± 2 cm⁻¹, 1060 ± 2 cm⁻¹, 858 ± 2 cm⁻¹ and 774 ± 2 cm⁻¹.

A process for the preparation of crystalline form I of Prasugrel hydrogensulfate comprising the steps of:
(a) heating a mixture of Prasugrel or a salt or a derivative thereof, sulfuric acid and optionally a solvent to a temperature of about 35 °C or more;
(b) reducing the temperature of the mixture to about 30 °C or below;
(c) adding seed crystals; and
(d) isolating crystalline form I of Prasugrel hydrogensulfate
is also subject matter of the present invention.

Another aspect the present invention relates to a pharmaceutical composition comprising crystalline form I of Prasugrel hydrogensulfate as defined above and optionally a pharmaceutically acceptable carrier.

The pharmaceutical composition can be used for inhibiting blood platelet aggregation. In particular, it can be employed for treating or preventing a disorder selected from the group consisting of thrombosis, embolism, coagulation induced vascular diseases and recurrence thereof and for use as an adjunct to percutaneous coronary intervention procedures. Furthermore, it can be used for preventing atherothrombotic events after myocardial infarction, after stroke, in patients having established peripheral arterial disease or in patients having Acute Coronary Syndrome.

Crystalline form I of Prasugrel hydrogensulfate shows high solubility within a broad pH range. This property enables the manufacture of finished dosage forms which, due to the low solubility of the prior art Prasugrel hydrochloride or Prasugrel maleate, could not be prepared easily, like liquid aqueous preparations for oral use (e.g. oral solutions, oral emulsions, oral suspensions, powders and granules for oral solutions and suspensions, oral drops, powder for oral drops, syrups, powders and granules for syrups), soluble tablets and parenteral preparations (e.g. injections, infusions, concentrates for injections or infusions, powders for injections or infusions, gels for injections, implants).

Other objects, features, advantages and aspects of the present invention will become apparent to those of skill from the following description.

### BRIEF DESCRIPTION OF THE DRAWINGS

Figure 1: X-ray powder diffraction pattern of crystalline form I of Prasugrel hydrogensulfate
Figure 2: Infrared spectrum of crystalline form I of Prasugrel hydrogensulfate

### DETAILED DESCRIPTION OF THE INVENTION

The present invention relates to crystalline form I of Prasugrel hydrogensulfate.

The chemical structure of Prasugrel hydrogensulfate is shown in Figure A.

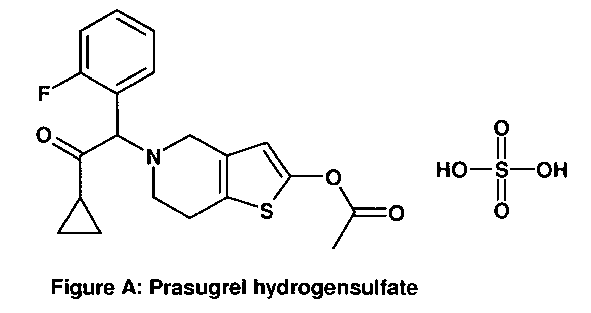

Prasugrel has an asymmetric carbon atom. The present invention covers the racemic form of Prasugrel hydrogensulfate as well as the individual stereoisomers and their mixtures.

Crystalline form I of Prasugrel hydrogensulfate can be prepared by
(a) heating a mixture of Prasugrel or a salt or a derivative thereof, sulfuric acid and optionally a solvent to a temperature of about 35 °C or more;
(b) reducing the temperature of the mixture to about 30 °C or below;
(c) adding seed crystals; and
(d) isolating crystalline form I of Prasugrel hydrogensulfate.

In step (a) a Prasugrel starting material is heated with sulfuric acid. The starting material can be Prasugrel itself or a salt thereof, in particular an acid addition salt of Prasugrel with an acid having a pKa of about 2 or more, such as the maleate salt, or another derivative thereof. Prasugrel, certain salts and derivatives can be prepared according to known procedures such as those mentioned in EP-A-542 411 or US-B-6,693,115.

Any suitable sulfuric acid can be used in step (a). Either diluted or concentrated sulfuric acid having a concentration in the range from about 5 to about 98 % can be employed. Preferably concentrated sulfuric acid, i.e. sulfuric acid having a concentration of about 95 to about 98 %, is used.

The ratio of Prasugrel to sulfuric acid can vary. Typically about 0.8 to about 5.0 equivalents, preferably about 0.9 to about 2.0 equivalents, most preferably about 1.2 to about 1.5 equivalents of sulfuric acid to 1 equivalent of Prasugrel (mol : mol) will be employed.

The Prasugrel starting material and the sulfuric acid are optionally in admixture with a solvent. Any type of solvent can be employed as long as it does not adversely effect the formation of the desired crystalline form I. Examples of suitable solvents are ketones such as acetone, methyl ethyl ketone or diethyl ketone and esters such as ethyl acetate, propyl acetate and butyl acetate. The solvent is preferably selected from ketones. Acetone is the most preferred solvent.

The Prasugrel starting material is preferably used in a concentration in the range from about 30 to about 500 g/I, more preferably about 100 to about 250 g/l, most preferably in a concentration of about 125 g/I if a solvent is employed.

The mixture of Prasugrel starting material, sulfuric acid and the optional solvent is heated to a temperature of about 35 °C or more until all of the Prasugrel starting material has dissolved. The temperature at which the Prasugrel starting material is dissolved is typically from about 35 °C to about 60 °C, preferably from about 35 °C to about 40 °C in order to minimize degradation. If desired, the mixture can be stirred during the dissolution step.

Pure crystalline form I of Prasugrel hydrogensulfate can be prepared in the following manner. After the Prasugrel starting material has been dissolved, the temperature of the solution is reduced to about 30 °C or below, preferably about -25 to about 25 °C, more preferably about 0 to about 25 °C. The cooling of the solution will typically take from about 30 minutes to about 300 minutes, preferably about 60 to about 180 minutes. The reaction mixture can be agitated during the cooling step, if necessary.

It was found that crystalline form I of Prasugrel hydrogensulfate does not form easily unless seed crystals are present. Those seed crystals were only obtained by serendipity after attempts to obtain a Prasugrel hydrogensulfate according to the general procedure of US 6,693,115 for the production of acid addition salts had failed.

In the process for the preparation of crystalline form I of Prasugrel hyrogensulfate, due to the cooling of the solution, crystals of form I of Prasugrel hydrogensulfate are able to grow after seeding. This is because at more elevated temperatures seed crystals might dissolve and Prasugrel hydrogensulfate might then remain in solution.

Seed crystals are added when the temperature of the solution has reached a value at which the seed crystals remain crystalline for a sufficiently long time, for example about 30°C or less, to facilitate crystallization. The seed crystals may be a pure crystalline form I of Prasugrel hydrogensulfate or may be Prasugrel hydrogensulfate seed crystals which can be prepared as set out in Example 1 of the present application.

The seed crystals obtained from example 1 are a mixture of amorphous Prasugrel hydrogensulfate and crystalline form I of Prasugrel hydrogensulfate..

After the temperature has been reduced to the desired temperature, the reaction mixture is left to stand at that temperature, so that crystallization can take place. The exact duration of the crystallization can vary. It will typically be from about 0.5 to about 48 hours, preferably from about 5 to about 24 hours. If desired, stirring can be conducted during this step, as long as the stirring is carried out in a manner which is gentle enough, so that it does not interfere with crystallization.

After the crystallization, crystalline form I of Prasugrel hydrogensulfate can be isolated from the mixture. Any conventional method such as filtration or evaporation of the solvent can be employed. If necessary, the crystals can be purified further by recrystallization.

The obtained crystals are mostly agglomerated but some larger lath-shaped crystals are also observed. The crystals show birefringence in polarized light which proves their crystallinity.

The molar ratio of Prasugrel to hydrogensulfate in the crystals is about 1:1.

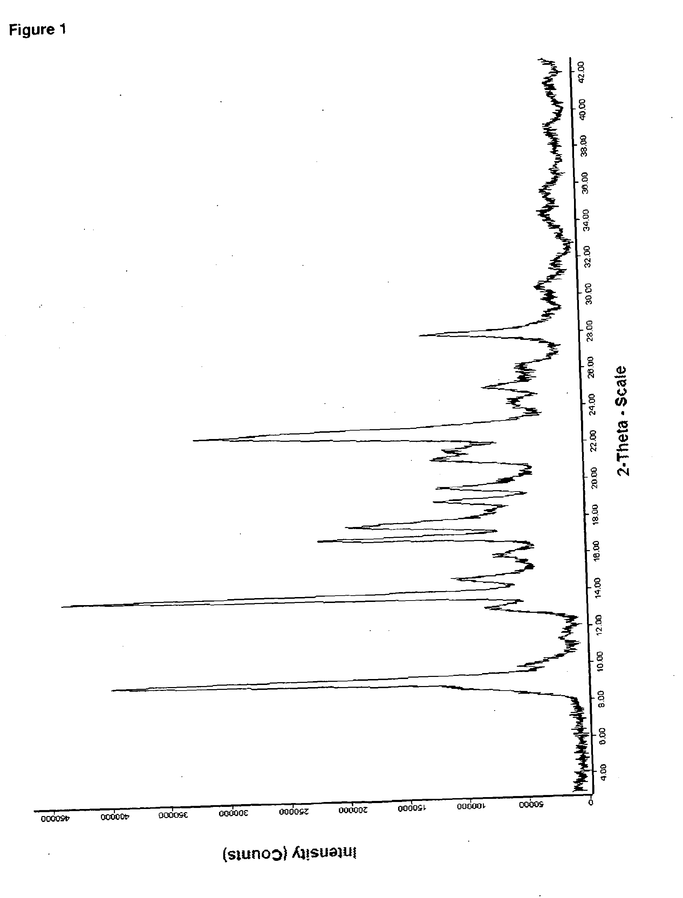

The crystalline form I of Prasugrel hydrogensulfate can be characterized by an X-ray powder diffraction pattern having peaks at 2-theta angles as shown in Table 1. A characteristic X-ray powder diffraction pattern of crystalline form I of Prasugrel hydrogensulfate is shown in Figure 1.

**Table 1: X-Ray Powder Diffraction (XRPD) pattern of crystalline form I of Prasugrel hydrogensulfate**

| Angle | relative intensity |
|---|---|
| [°2-theta ± 0.2°] | [%] |
| 9.2 | 91.0 |
| 13.1 | 19.0 |
| 13.9 | 100.0 |
| 14.8 | 25.1 |
| 16.0 | 17.1 |
| 17.0 | 50.8 |
| 17.7 | 45.4 |
| 18.9 | 28.4 |
| 19.7 | 27.7 |
| 21.2 | 28.7 |
| 22.7 | 73.7 |
| 25.1 | 18.2 |
| 28.0 | 30.0 |

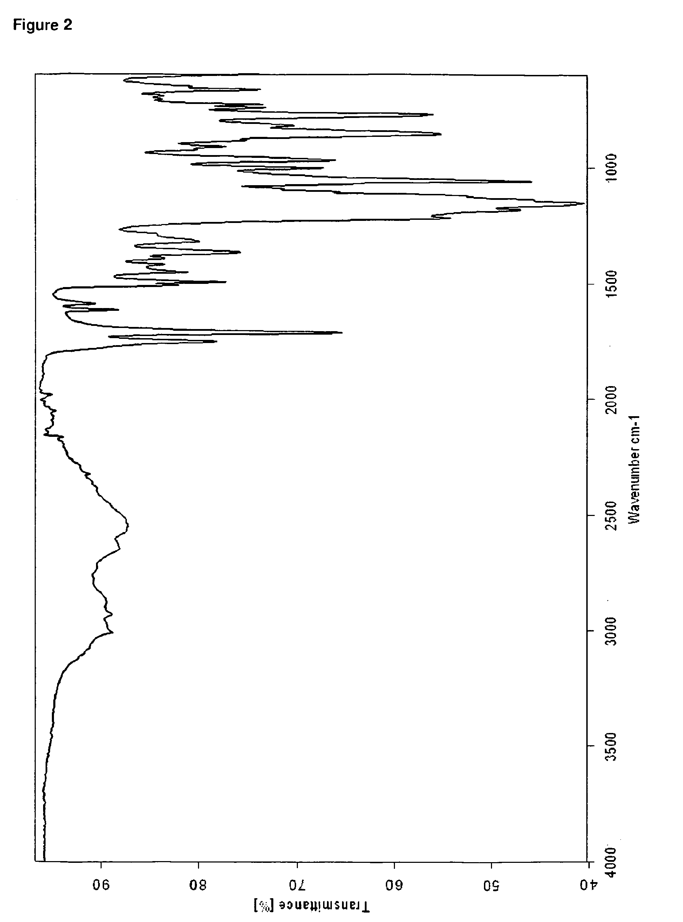

Crystalline form I of Prasugrel hydrogensulfate may be also characterized by an infrared spectrum having characteristic bands at 1751, 1712, 1495, 1153, 1060, 858 and 774 cm⁻¹. A usual deviation for these bands is ± 2 cm⁻¹. A typical IR spectrum is shown in Figure 2.

The present inventors have surprisingly found that Prasugrel hydrogensulfate possesses a higher solubility over a broad pH range from about pH 1.0 up to about pH 8.0, which reflects the pH-range in the stomach and the intestine in humans compared to other salts of Prasugrel, such as the hydrochloride or maleate salts. As bioavailability depends, among others, on solubility, an optimal active substance for oral application should show a high solubility in a broad pH-range. The solubility of various Prasugrel salts was determined according to the European Pharmacopoeia 6.0, 5.11 (2008) and is given in Table 2. Table 2 shows that the crystalline form I of Prasugrel hydrogensulfate is freely soluble in each of the different pH-media, whereas Prasugrel hydrochloride is only sparingly soluble at pH 1.2 and insoluble at pH 4.5 and in water. Prasugrel maleate is very slightly soluble at pH 1.2 and insoluble at pH 4.5 and in water.

**Table 2: Solubility of various Prasugrel acid addition salts**

| Prasugrel | Hydrochloric acid medium pH 1.2 | Acetate buffer pH 4.5 | water |
|---|---|---|---|
| hydrogensulfate | freely soluble | freely soluble | freely soluble |
| maleate | very slightly soluble | insoluble | insoluble |
| hydrochloride | sparingly soluble | insoluble | insoluble |

The crystalline form I of Prasugrel hydrogensulfate according to the present invention can be employed to treat or prevent any of the disorders which can be treated by Prasugrel or any of the other salts of Prasugrel. It is envisaged that it can also be employed to treat disorders which are indicated for the related compound Clopidogrel.

In particular, crystalline form I of Prasugrel hydrogensulfate can be used for treating or preventing a disorder selected from the group consisting of thrombosis, embolism, coagulation induced vascular diseases and recurrence thereof and for use as an adjunct to percutaneous coronary intervention procedures. Examples of coagulation induced vascular diseases and recurrence thereof are myocardial infarction, angina, pulmonary embolism, transient ischemic attack, deep vein thrombosis, thrombotic re-occlusion subsequent to a coronary intervention procedure, heart surgery or vascular surgery, peripheral vascular thrombosis, vascular diseases associated with diabetes mellitus, and/or Syndrome X (metabolic syndrome), heart failure, vascular complications of diabetes, and a disorder in which a narrowing of at least one coronary artery occurs. Preferably crystalline form I of Prasugrel hydrogensulfate can be used for preventing atherothrombotic events after myocardial infarction, after stroke, in patients having established peripheral arterial disease or in patients having Acute Coronary Syndrome. Prasugrel hydrogensulfate form I is a suitable form especially for acute indications mentioned in the last sentence where the anti-platelet action of the active substance should start as fast as possible. High solubility increases the dissolution rate which leads to faster absorption of the active substance into the blood and therefore to a faster onset of the anti-platelet activity.

Crystalline form I of Prasugrel hydrogensulfate can be administered alone or in combination with other pharmaceutically active compounds such as acetyl salicylic acid. The crystalline form I of Prasugrel hydrogensulfate and the other pharmaceutically active compound can be administered either simultaneously or sequentially.

The formulation of crystalline form I of Prasugrel hydrogensulfate is not particularly limited and it can be formulated according to known principles, e.g. either alone or together with pharmaceutically acceptable carriers, excipients, diluents and the like.

The crystalline form I of Prasugrel hydrogensulfate can be administered according to any appropriate route. Typically it will be administered orally or parenterally, preferably orally. Preferred formulations are liquid aqueous preparations for oral use (e.g. oral solutions, oral emulsions, oral suspensions, powders and granules for oral solutions and suspensions, oral drops, powder for oral drops, syrups, powders and granules for syrups), soluble tablets and parenteral preparations (e.g. injections, for example subcutaneous injections, infusions, concentrates for injections or infusions, powders for injections or infusions, gels for injections, implants).

Typical formulations and indications for Prasugrel are described, for example, in US 6,693,115, WO 2006/135605, WO 2007/024472, US 2007/0203157, EP-A-1 310 245, WO 97/17064, US 2007/0003628, WO 2005/048992, and WO 2007/113857 In those references relating to Clopidogrel, it is to be understood that Clopidogrel is to be replaced by the crystalline form I of Prasugrel hydrogensulfate according to the present invention. It is to be noted that these patents and patent applications are given as an example only and that this list is not to be considered exhaustive.

As mentioned above, compared to other salts of Prasugrel, such as the hydrochloride or maleate salts, the Prasugrel hydrogensulfate of the present invention possesses a higher solubility over a broad pH range from about pH 1.0 up to about pH 8.0, which reflects the pH-range in the stomach and the intestine in humans.

The present invention is illustrated in the following examples, which should not be construed as limiting.

### EXAMPLES

The powder diffractogram was collected on a Unisantis XMD 300 X-ray powder diffractometer with a position sensitive detector in parallel beam optics using the following acquisition conditions: tube anode: Cu, 40 kV, 0.8 mA; 3-43° theta/2-theta; simultaneous detection of regions of 10 ° per step with detector resolution 1024, counting time 300 seconds per step. A typical precision of the 2-theta values is in the range of ± 0.2° 2-theta. Thus a diffraction peak that appears at 5.0 2-theta can appear between 4.8 and 5.2° 2-theta on most X-ray diffractometers under standard conditions.

The infrared spectrum was collected on a diamond ATR cell with an Bruker Tensor 27 FTIR spectrometer with 4 cm⁻¹ resolution. A typical precision of the wavenumber values is in the range of ± 2 cm⁻¹. Thus an infrared peak that appears at 1716 cm⁻¹ can appear between 1714 and 1718 cm⁻¹ on most infrared spectrometers under standard conditions.

### Example 1: Preparation of seed crystals

1 ml ethyl acetate was added to 1.2 eq. (18 µl) concentrated sulfuric acid (95-97 %) and the solution was stirred at room temperature. After the addition of 100.0 mg Prasugrel a sticky coagulated mass of amorphous Prasugrel hydrogensulfate was obtained. The solvent was removed and the solid was dried under vacuum at room temperature for 2 hours. This material was added to a solution of 1.2 eq. (18 µl) concentrated sulfuric acid (95-97 %) and 100.0 mg Prasugrel in 800 µl acetone. The solution was stored without stirring for about 20 hours at -25 °C to grow white to off-white crystals. The solvent was removed and the crystals were dried under vacuum at room temperature to obtain a mixture of amorphous Prasugrel hydrogensulfate and crystalline form I of Prasugrel hydrogensulfate.

It should be noted that in an initial experiment the time required to obtain seed crystals was significantly longer, in the order of about three weeks. However, the procedure described in example 1 now provides seed crystals after the indicated 20 hour incubation.

Repeated cycles of the above procedure wherein a small amount of the obtained mixture was additionally added as a seed material to the Prasugrel / acetone / sulfuric acid solution further increased crystallinity of the obtained Prasugrel hydrogensulfate until 145.0 mg of Prasugrel hydrogensulfate form I seed crystals were obtained.

### Example 2: Preparation of form I of Prasugrel hydrogensulfate

250.0 mg Prasugrel and 1.2 eq. (44 µl) concentrated sulfuric acid (95 - 97 %) were dissolved in 2 ml acetone at 40 °C. The solution was slowly cooled down to room temperature and Prasugrel hydrogensulfate seed crystals obtained in Example 1 were added. After 17.5 hours stirring at room temperature, the precipitate was filtered off, washed with acetone and dried at room temperature under vacuum to obtain 199.1 mg (63 % yield) of crystalline form I of Prasugrel hydrogensulfate.

### Example 3: Preparation of form I of Prasugrel hydrogensulfate

200.0 mg Prasugrel maleate was slurried in 4 ml acetone at 60 °C. After the addition of 1.2 eq. (28 µl) concentrated sulfuric acid (95-97 %) a solution was obtained. The solution was then cooled down to 0-5 °C and Prasugrel hydrogensulfate seed crystals obtained in Example 1 were added. After 3 hours of stirring at 0-5 °C the precipitate was filtered off, washed with acetone and dried at room temperature under vacuum to obtain 96.9 mg (50 % yield) of crystalline form I of Prasugrel hydrogensulfate.

### Example 4: HPLC analysis

Assay calculated as Prasugrel hydrogensulfate: 97.3 %

| **HPLC conditions** | |
|---|---|
| Column | YMC-Hydrosphere C18, 150x4.6 mm, S-3 µm |
| Mobile phase A | H₂O/amidosulfonic acid (1000 g/3.884 g) |
| Mobile phase B | acetonitrile/H₂O/amidosulfon acid (588 g/250 g/3.884 g) |
| Injection volume | 7 µl |
| Flow rate | 0.8 ml/min |
| Oven temperature | 40 °C |
| Detection | UV 254 nm |

The Prasugrel hydrogensulfate of the present invention may be used in the following formulation examples, which should not be contrued in any way to be limiting.

The materials of the following formulations are to be combined before direct compression is performed to obtain 5 mg Prasugrel tablets (6.30 mg Prasugrel hydrogensulfate).

### Formulation 1

| | |
|---|---|
| Prasugrel hydrogensulfate | 6.30 mg (equivalent to 5.00 mg base) |
| Lactose monohydrate | 133.28 mg |
| Corn starch | 23.42 mg |
| Highly disperse SiO₂ | 2.50 mg |
| Stearic acid | 4.50 mg |
| Total | 170.00 mg |

### Formulation 2

| | |
|---|---|
| Prasugrel hydrogensulfate | 6.30 mg (equivalent to 5.00 mg base) |
| Mannitol | 109.86 mg |
| Cellulose microcrystalline | 46.84 mg |
| Highly disperse SiO₂ | 2.50 mg |
| Stearic acid | 4.50 mg |
| Total | 170.00 mg |

### Formulation 3

| | |
|---|---|
| Prasugrel hydrogensulfate | 6.30 mg (equivalent to 5.00 mg base) |
| Lactose anhydrous | 84.65 mg |
| Cellulose microcrystalline | 80.80 mg |
| Glyceryl dibehenate | 8.25 mg |
| Total | 180.00 mg |

### Formulation 4

| | |
|---|---|
| Prasugrel hydrogensulfate | 6.30 mg (equivalent to 5.00 mg base) |
| Lactose anhydrous | 70.89 mg |
| Cellulose microcrystalline | 67.07 mg |
| Glyceryl dibehenate | 8.25 mg |
| Pregelatinised starch | 27.49 mg |
| Total | 180.00 mg |

### Formulation 5

| | |
|---|---|
| Prasugrel hydrogensulfate | 6.30 mg (equivalent to 5.00 mg base) |
| Lactose anhydrous | 62.65 mg |
| Cellulose microcrystalline | 80.80 mg |
| Glyceryl dibehenate | 8.25 mg |
| Pregelatinised starch | 13.75 mg |
| Talc | 8.25 mg |
| Total | 180.00 mg |

### Formulation 6

| | |
|---|---|
| Prasugrel hydrogensulfate | 6.30 mg (equivalent to 5.00 mg base) |
| Lactose anhydrous | 76.40 mg |
| Cellulose microcrystalline | 67.05 mg |
| Glyceryl dibehenate | 8.25 mg |
| Hydroxypropyl cellulose | 13.75 mg |
| Talc | 8.25 mg |
| Total | 180.00 mg |

### Formulation 7

| | |
|---|---|
| Prasugrel hydrogensulfate | 6.30 mg (equivalent to 5.00 mg base) |
| Lactose anhydrous | 76.40 mg |
| Cellulose microcrystalline | 67.05 mg |
| Glyceryl dibehenate | 8.25 mg |
| Crospovidone | 13.75 mg |
| Talc | 8.25 mg |
| Total | 180.00 mg |

### Formulation 8

| | |
|---|---|
| Prasugrel hydrogensulfate | 6.30 mg (equivalent to 5.00 mg base) |
| Lactose anhydrous | 97.70 mg |
| Methylcellulose 15CPS | 34.00 mg |
| Crospovidone | 24.00 mg |
| Magnesium stearate | 7.60 mg |
| Colloidal silicon dioxide | 0.40 mg |
| Total | 170.00 mg |

### Formulation 9

| | |
|---|---|
| Prasugrel hydrogensulfate | 6.30 mg (equivalent to 5.00 mg base) |
| Lactose anhydrous | 97.70 mg |
| Methylcellulose 15CPS | 34.00 mg |
| Crospovidone | 24.00 mg |
| Calcium stearate | 7.60 mg |
| Colloidal silicon dioxide | 0.40 mg |
| Total | 170.00 mg |

### Formulation 10

| | |
|---|---|
| Prasugrel hydrogensulfate | 6.30 mg (equivalent to 5.00 mg base) |
| Lactose anhydrous | 97.70 mg |
| Methylcellulose 15CPS | 34.00 mg |
| Crospovidone | 24.00 mg |
| Zinc stearate | 7.60 mg |
| Colloidal silicon dioxide | 0.40 mg |
| Total | 170.00 mg |

### Formulation 11

| | |
|---|---|
| Prasugrel hydrogensulfate | 6.30 mg (equivalent to 5.00 mg base) |
| Lactose anhydrous | 97.70 mg |
| Methylcellulose 15CPS | 34.00 mg |
| Crospovidone | 24.0 mg |
| Sodium stearyl fumarate | 7.60 mg |
| Colloidal silicon dioxide | 0.40 mg |
| Total | 170.00 mg |

### Formulation 12

| | |
|---|---|
| Prasugrel hydrogensulfate | 6.30 mg (equivalent to 5.00 mg base) |
| Lactose anhydrous | 97.70 mg |
| Methylcellulose 15CPS | 34.00 mg |
| Crospovidone | 24.0 mg |
| Stearic acid | 17.60 mg |
| Colloidal silicon dioxide | 0.40 mg |
| Total | 180.00 mg |

## Claims

1. Crystalline form I of Prasugrel hydrogensulfate having an X-ray powder diffraction pattern comprising peaks at 2-theta angles of 9.2 ± 0.2°, 13.1 ± 0.2°, 13.9 ± 0.2°, 14.8 ± 0.2°, 16.0 ± 0.2°. 17.0 ± 0.2°, 17.7 ± 0.2°, 18.9 ± 0.2°, 19.7 ± 0,2°,21.2 ± 0.2°, 22.7 ± 0.2°, 25.1 ± 0.2° and 28.0 ± 0.2°.

2. The crystalline form I of Prasugrel hydrogensulfate according to claim 1 having an infrared spectrum comprising peaks at wavenumbers of 1751 ± 2 cm⁻¹, 1712 ± 2 cm⁻¹, 1495 ± 2 cm⁻¹, 1153 ± 2 cm⁻¹, 1060 ± 2 cm⁻¹, 858 ± 2 cm⁻¹ and 774 ± 2 cm⁻¹.

3. A process for the preparation of crystalline form I of Prasugrel hydrogensulfate comprising the steps of:
(a) heating a mixture of Prasugrel or a salt thereof, sulfuric acid and optionally a solvent to a temperature of about 35°C or more;
(b) reducing the temperature of the mixture to about 30 °C or below;
(c) adding seed crystals; and
(d) isolating crystalline form I of Prasugrel hydrogensulfate.

4. A pharmaceutical composition comprising crystalline form I of Prasugrel hydrogensulfate as defined in any one of claims 1 to 2 and optionally a pharmaceutically acceptable carrier.

5. Crystalline form I of Prasugrel hydrogensulfate as defined in any one of claims 1 to 2 for use as a medicament.

## Patentansprüche

1. Kristalline Form I von Prasugrel-Hydrogensulfat, das ein Röntgen-Pulverdiffraktionsmuster umfassend Peaks bei 2-Theta-Winkeln von 9,2 ± 0,2°, 13,1 ± 0,2°, 13,9 ± 0,2°, 14,8 ± 0,2°, 16,0 ± 0,2°, 17,0 ± 0,2°, 17,7 ± 0,2°, 18,9 ± 0,2°, 19,7 ± 0,2°, 21,2 ± 0,2°, 22,7 ± 0,2°, 25,1 ± 0,2° und 28,0 ± 0,2° aufweist.

2. Kristalline Form I von Prasugrel-Hydrogensulfat nach Anspruch 1, das ein Infrarotspektrum umfassend Peaks bei Wellenzahlen von 1751 ± 2 cm⁻¹, 1712 ± 2 cm⁻¹, 1495 ± 2 cm⁻¹, 1153 ± 2 cm⁻¹, 1060 ± 2 cm⁻¹, 858 ± 2 cm⁻¹ und 774 ± 2 cm⁻¹, aufweist.

3. Verfahren für die Herstellung von kristalliner Form I von Prasugrel-Hydrogensulfat, umfassend die Schritte:
(a) Erhitzen einer Mischung von Prasugrel oder einem Salz davon, Schwefelsäure und gegebenenfalls einem Lösungsmittel auf eine Temperatur von etwa 35 °C oder mehr;
(b) Reduzieren der Temperatur der Mischung auf etwa 30 °C oder weniger;
(c) Zufügen von Impfkristallen; und
(d) Isolieren von kristalliner Form I von Prasugrelhydrogensulfat.

4. Pharmazeutische Zusammensetzung umfassend kristalline Form I von Prasugrel-Hydrogensulfat wie in einem beliebigen der Ansprüche 1 bis 2 definiert und gegebenenfalls einen pharmazeutisch verträglichen Träger.

5. Kristalline Form I von Prasugrel-Hydrogensulfat wie in einem beliebigen der Ansprüche 1 bis 2 definiert zur Verwendung als ein Medikament.

## Revendications

1. Forme cristalline I d'hydrogénosulfate de Prasugrel ayant un diagramme de diffraction des rayons X sur poudre comprenant des pics aux angles 2-thêta de 9,2 ± 0,2° ; 13,1 ± 0,2° ; 13,9 ± 0,2° ; 14,8 ± 0,2° ; 16,0 ± 0,2° ; 17,0 ± 0,2° ; 17,7 ± 0,2° ; 18,9 ± 0,2° ; 19,7 ± 0,2° ; 21,2 ± 0,2° ; 22,7 ± 0,2° ; 25,1 ± 0,2° et 28,0 ± 0,2°.

2. Forme cristalline I d'hydrogénosulfate de Prasugrel selon la revendication 1 possédant un spectre infrarouge comprenant des pics aux nombres d'ondes de 1 751 ± 2 cm⁻¹, 1 712 ± 2 cm⁻¹, 1 495 ± 2 cm⁻¹, 1 153 ± 2 cm⁻¹, 1 060 ± 2 cm⁻¹, 858 ± 2 cm⁻¹ et 774 ± 2 cm⁻¹.

3. Procédé pour la préparation de la forme cristalline I d'hydrogénosulfate de Prasugrel comprenant les étapes de :
(a) chauffage d'un mélange de Prasugrel ou d'un sel de celui-ci, d'acide sulfurique et éventuellement d'un solvant à une température d'environ 35°C ou plus ;
(b) réduction de la température du mélange à environ 30°C ou moins ;
(c) addition de germes cristallins ; et
(d) isolation de la forme cristalline I d'hydrogénosulfate de Prasugrel.

4. Composition pharmaceutique comprenant la forme cristalline I d'hydrogénosulfate de Prasugrel telle que définie dans l'une quelconque des revendications 1 et 2 et éventuellement un véhicule pharmaceutiquement acceptable.

5. Forme cristalline I d'hydrogénosulfate de Prasugrel telle que définie dans l'une quelconque des revendications 1 et 2 pour une utilisation en tant que médicament.
